(19) Europäisches Patentamt
European Patent Office
Office européen des brevets

(11) EP 3 461 472 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**27.12.2023 Patentblatt 2023/52**

(45) Hinweis auf die Patenterteilung:
**24.02.2021 Patentblatt 2021/08**

(21) Anmeldenummer: **18198207.5**

(22) Anmeldetag: **02.10.2018**

(51) Internationale Patentklassifikation (IPC):
***A61K 8/44*** *(2006.01)* ***A61K 8/46*** *(2006.01)*
***A61K 8/86*** *(2006.01)* ***A61Q 19/10*** *(2006.01)*

(52) Gemeinsame Patentklassifikation (CPC):
**A61Q 19/10; A61K 8/442; A61K 8/466; A61K 8/86;**
A61K 2800/48; A61K 2800/596

(54) **SENSITIVES HAUTREINIGUNGSMITTEL MIT VERBESSERTER REINIGUNGSWIRKUNG**
SENSITIVE SKIN CLEANSER WITH ENHANCED CLEANING EFFECT
PRODUITS DE NETTOYAGE DE LA PEAU SENSIBLE À EFFET NETTOYANT AMÉLIORÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2017 EP 17194388**

(43) Veröffentlichungstag der Anmeldung:
**03.04.2019 Patentblatt 2019/14**

(73) Patentinhaber: **Peter Greven Physioderm GmbH**
**53902 Bad Münstereifel (DE)**

(72) Erfinder:
- **Stolz, Hermann Josef**
  **53902 Bad Münstereifel (DE)**
- **Nolte, Bert**
  **53902 Bad Münstereifel (DE)**
- **Börnicke, Robert**
  **53902 Bad Münstereifel (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**WO-A1-2015/018667** **WO-A2-2005/051341**
**WO-A2-2008/138708** **WO-A2-2015/018567**
**US-A1- 2016 100 574**

Bemerkungen:
Die Akte enthält technische Angaben, die nach dem Eingang der Anmeldung eingereicht wurden und die nicht in dieser Patentschrift enthalten sind.

EP 3 461 472 B2

**Beschreibung**

Die Erfindung betrifft Hautreinigungsmittel sowie ihre Verwendung.

Hauterkrankungen gehören zu den zehn häufigsten Berufserkrankungen. Eine möglichst schonende Reinigung der Haut trägt besonders im Berufsalltag zum Erhalt eines guten Hautzustandes entscheidend bei.

Pastöse reibemittelhaltige Hautreinigungsmittel werden in der Industrie zum Entfernen von hartnäckigen Verschmutzungen, wie Ölen, Rußen, Schmierfetten, Bitumen, Lacken usw. eingesetzt.

Einerseits nimmt durch den technologischen Fortschritt der Grad der Verschmutzung in vielen Anwendungsbereichen von Hautreinigungsmitteln ab. Dies führt dazu, dass teilweise auf Reibemittel, die einen Provokationsfaktor für Dermatosen darstellen, verzichtet oder deren Einsatz zumindest reduziert werden kann. Andererseits stellen solche Produkte aber erhöhte Anforderungen an die Reinigungseigenschaften, da Hautreinigungsmittel bei häufiger Anwendung auch zu einer Entfettung und damit Austrocknung der Haut führen können. In anderen Bereichen kann auch heutzutage auf Reibemittel nicht verzichtet werden.

DE 4038076 A1 offenbart die Verwendung eines Reibemittels aus natürlichen Schalen und/oder Kernen in feiner Verteilung, das mit einem Bleichmittel behandelt wurde. Offenbart wird auch die Verwendung von Natriumlaurylsulfat in Kombination mit Natriumalkylbenzolsulfonat sowie von Mischungen aus Ethersulfat und Betain. Der Hautverträglichkeit wird allenfalls durch rückfettende Substanzen Rechnung getragen.

DE 197 48 921 A1 offenbart wasserhaltige flüssige pasten- oder cremeförmige Handreinigungsmittel, insbesondere Grobhandreiniger mit einem Reibemittel, die gekennzeichnet sind durch einen Gehalt an 10 bis 30 Gew.-% wenigstens eines pflanzlichen Öls aus der Gruppe der Triglyceride, gesättigten und/oder ungesättigten Fettsäuren, an 10 bis 30 Gew.-% wenigstens eines Tensids aus der Gruppe der Fettalkoholethoxylate, Fettalkoholethersulfate und/oder Ricinusölsulfonate, 10 bis 65 Gew.-% Wasser, jeweils bezogen auf die Zusammensetzung der Reinigungsmittel. Bevorzugt werden Fettalkoholethoxylate mit einer Kettenlänge des Alkoholanteils von 8 bis 18 C-Atomen und einem Ethoxylierungsgrad von 1 - 8, bevorzugt 6. Da die Tenside selbst keine hohe Hautverträglichkeit aufweisen, weisen die Formulierungen zu deren Erhöhung hohe Anteile an Ölen auf, wodurch Schaumverhalten und Reinigungseigenschaften teilweise negativ beeinflusst werden

EP 1 152 051 A2 offenbart wässrige Reinigungsmittel, enthaltend Fettsäurealkylester und Emulgatoren. Auf die Eigenschaften der nichtionogenen Tenside im Einzelnen oder der Tensidkombinationen wird nicht eingegangen.

US 2016/0100574 A1 offenbart Glycerylether von aromatischen Verbindungen als Konservierungsmittel für u.a. kosmetische Zusammensetzungen.

WO 2005/051341 A2 offenbart kosmetsiche Zusammensetzungen, die Vitamin A, Vitamin E und Aloe enthalten.

Grundsätzlich besteht auch bei den Formulierungen mit Reibemitteln, auf die in vielen Anwendungsbereichen noch nicht verzichtet werden kann, weiter Bedarf an Produkten mit hervorragenden Reinigungseigenschaften bei möglichst guter Hautverträglichkeit.

Aufgabe der vorliegenden Erfindung war es, ein Hautreinigungsmittel zu entwickeln, das die vorgenannten Nachteile des Standes der Technik zumindest teilweise überwindet, insbesondere ein Hautreinigungsmittel mit sowohl guter Reinigungswirkung als auch verbesserter Hautverträglichkeit.

Hierzu wurden in mehreren Studien einzelne Tenside, aber auch Tensidkombinationen auf ihre Eigenschaften hin untersucht. Als Standards dienten hierbei zum Einen 0,5%-iges Natriumlaurylsulfat (*sodium lauryl sulfate,* SLS), zum Anderen ein Hautreiniger für Atopiker, der eine gute Reinigungswirkung bei geringem irritativem Potential zeigt. Die Studien umfassten mehrere Tests der Hautverträglichkeit sowie der Reinigungswirkung, wie in den nachfolgenden Beispielen beschrieben.

Gelöst wird die Aufgabe durch ein Hautreinigungsmittel gemäß Anspruch 1.

Das erfindungsgemäße Hautreinigungsmittel enthält also sowohl mindestens ein nichtionogenes Tenside der Formel I als auch Cotenside. Das nichtionogene Tensid besteht aus dem Kondensationsprodukt eines Fettalkohols mit 5 bis 40 C-Atomen und einem Polyethylenglykol mit 5 bis 50 Glykolmonomeren, wobei der Fettalkohol ein lineare oder verzweigte, substituierte oder unsubstituierte, gesättigte oder ungesättigte Kohlenwasserstoffkette aufweist.

Das Cotensid ist ausgewählt aus Kombinationen von Sulfosuccinaten und Betainen.

Sulfosuccinate sind Mono- oder Diester der Sulfobernsteinsäure. Typische Vertreter sind Laurethsulfosuccinate, Dioctylsulfosuccinate, Dihexylsulfosuccinate und Laurylsulfosuccinat.

Betaine sind amphotere Tenside umfassend eine quatäre Ammoniumgruppe und ein Carboxylat und einen unpolaren Teil. Da der Stickstoff vier organische Reste trägt, kann die Ladung im Molekül nicht ausgeglichen werden. Typische Vertreter sind Cocamidopropylbetain, Decylbetain, Laurylbetain, Oleamido-Propylbetain und Hexyldecylbetain.

Sowohl das nichtionogene Tensid als auch das Cotensid sind jeweils in einer Menge von 2 bis 40 Gew.-% bezogen auf die Gesamtmenge des Hautreinigungsmittels enthalten, wobei beim Cotensid gegebenenfalls vorhandene Gegenionen nicht mit einberechnet werden.

Weiterhin umfasst das erfindungsgemäße Hautreinigungsmittel 0,1 bis 20 Gew.-% Verdickungsmittel, bezogen auf die Gesamtmenge an Hautreinigungsmittel, sowie Wasser.

In einer Ausführungsform umfasst das Hautreinigungsmittel keine Fettsäurealkylester.

Erfindungsgemäß ist eines der beiden Cotenside ein Sulfosuccinat und eines der beiden Cotenside ein Betain, bevorzugt ist eine Ausführungsform wobei es sich bei den beiden Cotensiden um Cocamidopropyl Betaine und Disodium Laureth Sulfosuccinate handelt.

Überraschenderweise ergibt sich bei der Kombination von nichtionogenen Tensiden mit zwei Cotensiden trotz Erhöhung des Gesamtaktivgehaltes und damit verbundener erhöhter Reinigungswirkungeine gleichbleibende Hautverträglichkeit.

In einer bevorzugten Ausführungsform beträgt die Menge aller nichtionogenen Tenside mit der allgemeinen Formel I 2 bis 20 Gew.-%, bevorzugt 2 bis 15 Gew.-%, mehr bevorzugt 2 bis 10 Gew.-%, noch mehr bevorzugt 3 bis 9 Gew.-%, jeweils bezogen auf die Gesamtmenge an Hautreinigungsmittel.

In einer weiteren Ausführungsform beträgt die Menge aller Cotenside 2 bis 20 Gew.-%, bevorzugt 2 bis 15 Gew.-%, mehr bevorzugt 2 bis 10 Gew.-%, noch mehr bevorzugt 2 bis 7 Gew.-%, berechnet jeweils ohne Gegenionen bezogen auf die Gesamtmenge an Hautreinigungsmittel.

In einer Ausführungsform weist das Hautreinigungsmittel eine Gesamtmenge an nichtionogenen Tensiden der allgemeinen Formel I und Cotensiden von 4 bis 25 Gew.-%, bevorzugt 4 bis 20 Gew.-%, mehr bevorzugt 5 bis 16 Gew.-%, jeweils berechnetohne Gegenionen bezogen auf die Gesamtmenge des Hautreinigungsmittels, auf. Die angegebenen Mengenangaben beziehen sich hier also auf die Summen aller nichtionogenen Tenside und Cotenside, wobei deren Gegenionen nicht einbezogen werden.

In einer bevorzugten Ausführungsform weist R in der Formel I des nichtionogenen Tensids 8 bis 30, bevorzugt 10 bis 24, am meisten bevorzugt 12 bis 18 C-Atome auf.

In einer weiteren bevorzugten Ausführungsform beträgt n in der Formel I des nichtionogenen Tensids 8 bis 50, bevorzugt 10 bis 40, mehr bevorzugt 14 bis 30, am meisten bevorzugt 15 bis 25.

Im Folgenden bezeichnet $\overline{n}$ das stoffmengengewichtete Mittel des n der Formel I, d.h. das stoffmengengewichtete Mittel der Anzahl der Ethylenglykoluntereinheiten in den nichtionogenen Tensiden der Formel I.

In einer Ausführungsform mit R aufweisend 5 bis 40 C-Atome, im stoffmengengewichteten Mittel aufweisend 12 bis 18 C-Atome, beträgt n 8 bis 50 und $\overline{n}$ 10 bis 40, 14 bis 30, 15 bis 25 oder 20 bis 25.

In einer Ausführungsform mit R aufweisend 8 bis 30 C-Atome, im stoffmengengewichteten Mittel aufweisend 12 bis 18 C-Atome, beträgt n 8 bis 50 und $\overline{n}$ 10 bis 40, 14 bis 30, 15 bis 25 oder 20 bis 25.

In einer Ausführungsform mit R aufweisend 10 bis 24 C-Atome, im stoffmengengewichteten Mittel aufweisend 12 bis 18 C-Atome, beträgt n 8 bis 50 und $\overline{n}$ 10 bis 40, 14 bis 30, 15 bis 25 oder 20 bis 25.

In einer Ausführungsform mit R aufweisend 12 bis 18 C-Atome, im stoffmengengewichteten Mittel aufweisend 12 bis 14 C-Atome, beträgt n 8 bis 50 und $\overline{n}$ 10 bis 40, 14 bis 30, 15 bis 25 oder 20 bis 25.

In einer Ausführungsform mit R aufweisend 12 bis 18 C-Atome, im stoffmengengewichteten Mittel aufweisend 14 bis 16 C-Atome, beträgt n 8 bis 50 und $\overline{n}$ 10 bis 40, 14 bis 30, 15 bis 25 oder 20 bis 25.

In einer Ausführungsform mit R aufweisend 5 bis 40 C-Atome, im stoffmengengewichteten Mittel aufweisend 12 bis 18 C-Atome, beträgt n 10 bis 40 und $\overline{n}$ 14 bis 30, 15 bis 25 oder 20 bis 25.

In einer Ausführungsform mit R aufweisend 8 bis 30 C-Atome, im stoffmengengewichteten Mittel aufweisend 12 bis 18 C-Atome, beträgt n 10 bis 40 und $\overline{n}$ 14 bis 30, 15 bis 25 oder 20 bis 25.

In einer Ausführungsform mit R aufweisend 10 bis 24 C-Atome, im stoffmengengewichteten Mittel aufweisend 12 bis 18 C-Atome, beträgt n 10 bis 40 und $\overline{n}$ 14 bis 30, 15 bis 25 oder 20 bis 25.

In einer Ausführungsform mit R aufweisend 12 bis 18 C-Atome, im stoffmengengewichteten Mittel aufweisend 12 bis 14 C-Atome, beträgt n 10 bis 40 und $\overline{n}$ 14 bis 30, 15 bis 25 oder 20 bis 25.

In einer Ausführungsform mit R aufweisend 12 bis 18 C-Atome, im stoffmengengewichteten Mittel aufweisend 14 bis 16 C-Atome, beträgt n 10 bis 40 und $\overline{n}$ 14 bis 30, 15 bis 25 oder 20 bis 25.

In einer Ausführungsform mit R aufweisend 5 bis 40 C-Atome, im stoffmengengewichteten Mittel aufweisend 12 bis 18 C-Atome, beträgt n 14 bis 30 und $\overline{n}$ 15 bis 25 oder 20 bis 25.

In einer Ausführungsform mit R aufweisend 8 bis 30 C-Atome, im stoffmengengewichteten Mittel aufweisend 12 bis 18 C-Atome, beträgt n 14 bis 30 und $\overline{n}$ 15 bis 25 oder 20 bis 25.

In einer Ausführungsform mit R aufweisend 10 bis 24 C-Atome, im stoffmengengewichteten Mittel aufweisend 12 bis 18 C-Atome, beträgt n 14 bis 30 und $\overline{n}$ 15 bis 25 oder 20 bis 25.

In einer Ausführungsform mit R aufweisend 12 bis 18 C-Atome, im stoffmengengewichteten Mittel aufweisend 12 bis 14 C-Atome, beträgt n 14 bis 30 und $\overline{n}$ 15 bis 25 oder 20 bis 25.

In einer Ausführungsform mit R aufweisend 12 bis 18 C-Atome, im stoffmengengewichteten Mittel aufweisend 14 bis 16 C-Atome, beträgt n 14 bis 30 und $\overline{n}$ 15 bis 25 oder 20 bis 25.

In einer Ausführungsform mit R aufweisend 5 bis 40 C-Atome, im stoffmengengewichteten Mittel aufweisend 12 bis 18 C-Atome, beträgt n 15 bis 25 und $\overline{n}$ 20 bis 25.

In einer Ausführungsform mit R aufweisend 8 bis 30 C-Atome, im stoffmengengewichteten Mittel aufweisend 12 bis 18 C-Atome, beträgt n 15 bis 25 und $\overline{n}$ oder 20 bis 25.

In einer Ausführungsform mit R aufweisend 10 bis 24 C-Atome, im stoffmengengewichteten Mittel aufweisend 12 bis 18 C-Atome, beträgt n 15 bis 25 und n 20 bis 25.

In einer Ausführungsform mit R aufweisend 12 bis 18 C-Atome, im stoffmengengewichteten Mittel aufweisend 12 bis 14 C-Atome, beträgt n 15 bis 25 und n 20 bis 25.

In einer Ausführungsform mit R aufweisend 12 bis 18 C-Atome, im stoffmengengewichteten Mittel aufweisend 14 bis 16 C-Atome, beträgt n 15 bis 25 und $\bar{n}$ 20 bis 25.

In einer Ausführungsform mit n betragend 8 bis 50, $\bar{n}$ betragend 15 bis 25, weist R 5 bis 40 C-Atome, im stoffmengengewichteten Mittel 8 bis 30, 10 bis 24, 12 bis 18 oder 12 bis 14 C-Atome auf.

In einer Ausführungsform mit n betragend 10 bis 40, $\bar{n}$ betragend 15 bis 25, weist R 5 bis 40 C-Atome, im stoffmengengewichteten Mittel 8 bis 30, 10 bis 24, 12 bis 18 oder 12 bis 14 C-Atome auf.

In einer Ausführungsform mit n betragend 14 bis 30, $\bar{n}$ betragend 15 bis 25, weist R 5 bis 40 C-Atome, im stoffmengengewichteten Mittel 8 bis 30, 10 bis 24, 12 bis 18 oder 12 bis 14 C-Atome auf.

In einer Ausführungsform mit n betragend 15 bis 25, $\bar{n}$ betragend 20 bis 25, weist R 5 bis 40 C-Atome, im stoffmengengewichteten Mittel 8 bis 30, 10 bis 24, 12 bis 18 oder 12 bis 14 C-Atome auf.

In einer Ausführungsform mit n betragend 8 bis 50, $\bar{n}$ betragend 15 bis 25, weist R 8 bis 30 C-Atome, im stoffmengengewichteten Mittel 10 bis 24, 12 bis 18 oder 12 bis 14 C-Atome auf.

In einer Ausführungsform mit n betragend 10 bis 40, $\bar{n}$ betragend 15 bis 25, weist R 8 bis 30 C-Atome, im stoffmengengewichteten Mittel 10 bis 24, 12 bis 18 oder 12 bis 14 C-Atome auf.

In einer Ausführungsform mit n betragend 14 bis 30, n betragend 15 bis 25, weist R 8 bis 30 C-Atome, im stoffmengengewichteten Mittel 10 bis 24, 12 bis 18 oder 12 bis 14 C-Atome auf.

In einer Ausführungsform mit n betragend 15 bis 25, n betragend 20 bis 25, weist R 8 bis 30 C-Atome, im stoffmengengewichteten Mittel 10 bis 24, 12 bis 18 oder 12 bis 14 C-Atome auf.

In einer Ausführungsform mit n betragend 8 bis 50, n betragend 15 bis 25, weist R 10 bis 24 C-Atome, im stoffmengengewichteten Mittel 12 bis 18 oder 12 bis 14 C-Atome auf.

In einer Ausführungsform mit n betragend 10 bis 40, n betragend 15 bis 25, weist R 10 bis 24 C-Atome, im stoffmengengewichteten Mittel 12 bis 18 oder 12 bis 14 C-Atome auf.

In einer Ausführungsform mit n betragend 14 bis 30, $\bar{n}$ betragend 15 bis 25, weist R 10 bis 24 C-Atome, im stoffmengengewichteten Mittel 12 bis 18 oder 12 bis 14 C-Atome auf.

In einer Ausführungsform mit n betragend 15 bis 25, $\bar{n}$ betragend 20 bis 25, weist R 10 bis 24 C-Atome, im stoffmengengewichteten Mittel 12 bis 18 oder 12 bis 14 C-Atome auf.

In einer Ausführungsform mit n betragend 8 bis 50, n betragend 15 bis 25, weist R 12 bis 18 C-Atome, im stoffmengengewichteten Mittel 12 bis 14 C-Atome auf.

In einer Ausführungsform mit n betragend 10 bis 40, n betragend 15 bis 25, weist R 12 bis 18 C-Atome, im stoffmengengewichteten Mittel 12 bis 14 C-Atome auf.

In einer Ausführungsform mit n betragend 14 bis 30, $\bar{n}$ betragend 15 bis 25, weist R 12 bis 18 C-Atome, im stoffmengengewichteten Mittel 12 bis 14 C-Atome auf.

In einer Ausführungsform mit n betragend 15 bis 25, n betragend 20 bis 25, weist R 12 bis 18 C-Atome, im stoffmengengewichteten Mittel 12 bis 14 C-Atome auf.

In einer Ausführungsform ist R in der Formel I des nichtionogenen Tensids eine ungesättigte oder verzweigte Kohlenwasserstoffkette.

In einer weiteren Ausführungsform ist R in der Formel I des nichtionogenen Tensids eine gesättigte, unverzweigte Kohlenwasserstoffkette, bevorzugt eine gesättigte, unverzweigte Kohlenwasserstoffkette mit 10 bis 24 C-Atomen, mehr bevorzugt eine gesättigte, unverzweigte Kohlenwasserstoffkette mit 12 bis 18 C-Atomen. Am meisten bevorzugt ist das nichtionogene Tensid Laureth-23.

In einer weiteren bevorzugten Ausführungsform weist das nichtionogene Tensid ein R mit 12 bis 18 C-Atomen und ein n von 14 bis 30, mehr bevorzugt ein R mit 12 bis 18 C-Atomen und ein n von 15 bis 25 auf.

In einer Ausführungsform umfasst das Hautreinigungsmittel weitere Inhaltsstoffe, zum Beispiel solche ausgewählt aus der Gruppe bestehend aus Stellmitteln, Rückfettern, Parfüms, Reibemittel, Konservierungsmitteln, Farbstoffen, Konditionierern, Feuchthaltemitteln und Kombinationen daraus.

In einer Ausführungsform umfasst das Hautreinigungsmittel weiterhin Reibemittel, wie Polyethylenpartikel, Polyurethanpartikel, Silikate, Biopolymere, Bimsmehl, Kork, Glaskörper, Wachskörper, natürliche Kerne, Kernmehle wie Olivenkernmehl, Schalenmehle, Holzmehle, Lignin, Ligninderivate, Cellulose, Cellulosederivate oder Kombinationen daraus. Besonders bevorzugt sind hierbei Olivenkernmehl und Kombinationen mit diesem.

In einer weiteren Ausführungsform umfasst das Hautreinigungsmittel weiterhin Überfettungsmittel, z.B. Glyceryloleate, Cetylpalmitate, Lanolin, natürliche Pflanzenöle, Squalene oder Mischungen von Alkyl- und/oder Alkenyloligoglykosiden und Partialglyceriden, oder Kombinationen daraus.

In einer weiteren Ausführungsform umfasst das Hautreinigungsmittel weiterhin Feuchthaltemittel (NMF), zum Beispiel Allantoin, Aminosäuren, Glycerin, Polyole, Harnstoff, Hyaluronsäure, Lactate, Lysozym, Serin, Vitamin A, Vitamin B, Vitamin C, hydrolysierte Pflanzenproteine, D-Panthenol oder Kombinationen daraus.

In einer bevorzugten Ausführungsform umfasst das Verdickungsmittel Bentonite, Xanthane, Acrylate, Alginate, Cellulose-Ether, Carrageen, oder Kombinationen daraus.

In einer weiteren bevorzugten Ausführungsform weist das Hautreinigungsmittel eine Viskosität bei 25 °C von 1000 bis 50000 mPas auf.

Bevorzugt ist weiterhin ein Hautreinigungsmittel, das bei einer Gesamtmenge an nichtionogenen Tensiden und Cotensiden von 8 Gew.-%, ohne Gegenionen bezogen auf die Gesamtmenge des Hautreinigungsmittels, einen Reinigungsfaktor von mindestens 50%, bevorzugt mindestens 55%, mehr bevorzugt mindestens 58%, am meisten bevorzugt mindestens 60%, aufweist, gemessen anhand einer Verschmutzung mit Modellschmutz bestehend (nach INCI) aus

- 54,0 Gew.-% Paraffinum Liquidum
- 18,1 Gew.-% Petrolatum
- 18,1 Gew.-% Lanolin
- 5,4 Gew.-% CI 77266 (Lampenruß)
- 3,6 Gew.-% CI77266 (Graphit)
- 0,8 Gew.-% CI 77499 (Eisenoxid), jeweils bezogen auf die Gesamtmenge des Modellschmutzes, und anhand einer Hautwaschung mit dem Osnabrücker Hautwaschapparat gemäß Sonsmann FK, et al. Standardization of skin cleansing in vivo: part I. Development of an Automated Cleansing Device (ACiD) Skin Res Technol 2014, 20, 228-38.

Eine Ausführungsform der Erfindung ist die Verwendung des erfindungsgemäßen Hautreinigungsmittels zur Hautreinigung.

Die Erfindung wird durch die nachstehenden Beispiele näher erläutert. Alle Angaben sind Gew-%, soweit nicht anders angegeben.

**Beispiel 1: Bestimmung der Hautverträglichkeit**

Die Hautverträglichkeit wurde anhand einer optischen Beurteilung (*visual score,* VS), einer Messung des transepidermalen Wasserverlusts (*transepidermal water loss,* TEWL) und einer Messung der Hautrötung (Faktor a) beurteilt. Es wurden die Rangfolgenlisten der drei genannten Tests ermittelt und diese zu der Rangfolge (RS) zusammengefasst:

$$RS = \text{Rangplatz Parameter VS} + \text{Rangplatz } a^* + \text{Rangplatz TEWL}$$

Die Reinigungswirkung erfolgte gemäß einem von Schrader und Rohr entwickelten Verfahren (Schrader K, Rohr M (1996): Methods for measuring the skin-cleansing effect of surfactants in comparison with skin roughness and compatibility. Clin Dermatol. 14(1):57-65.) Als Modellschmutz, vergleichbar mit der Verschmutzung durch Altöl, diente die Formulierung gemäß Tabelle 1.

**Tabelle 1**

| Inhaltsstoffe nach INCI* | Gew.-% |
|---|---|
| PARAFFINUM LIQUIDUM | 54,0 |
| PETROLATUM | 18,1 |
| LANOLIN | 18,1 |
| CI 77266 (Lampenruß) | 5,4 |
| CI 77266 (Graphit) | 3,6 |
| CI 77499 (Eisenoxid) | 0,8 |
| **international nomenclature of cosmetic ingredients* | |

Der Modellschmutz wurde nach einem definierten Verfahren auf Arealen des Unterarms aufgetragen. Die Beurteilung der Reinigungswirkung erfolgte durch optische Messung und wird als Reinigungsfaktor (RF) ausgedrückt:

$$RF\,[\%] = \frac{(W-L)\times 100}{A-L},$$

mit

A = Farbe des Testareals vor der Anschmutzung
L = Farbe des Testareals nach der Anschmutzung
W = Farbe des Testareals nach der Waschung.

Die Waschungen wurden mit dem Osnabrücker Hautwaschapparat (OHWA; Sonsmann FK, Strunk M, Gediga K et al. Standardization of skin cleansing in vivo: part I. Development of an Automated Cleansing Device (ACiD). Skin Res Technol 2014; 20: 228-38) durchgeführt und die Formulierungen mit einem repetitiven offenen Irritationstest (RIT) mittels des OHWA auf ihre Hautverträglichkeit untersucht.

Final wurden die Formulierungen mit einem repetitiven offenen Irritationstest mittels des Osnabrücker Hautwaschapparat (OHWA) auf ihre Hautverträglichkeiten hin untersucht (Sonsmann et al. 2014). Es wurden Aktivgehalte der Tenside von 8,5% getestet.

Als Standards dienten hierbei zum Einen Natriumlaurylsulfat (*sodium lauryl sulfate*, SLS) mit einem Aktivgehalt von 0,5%, zum Anderen ein Hautreiniger für Atopiker, der eine gute Reinigungswirkung bei geringem irritativem Potential zeigt.

Die Zusammensetzung des Hautreinigers für Atopiker ergibt sich aus der Tabelle 2.

**Tabelle 2**

| Inhaltsstoffe nach INCI | Gew.-% | Aktivgehalt [%] |
|---|---|---|
| DISODIUM LAURETH SULFOSUCCINATE | 20 | 8 |
| DISODIUM COCOAMPHODIACETATE | 10 | 4 |
| POTASSIUM COCOYL HYDROLYZED COLLAGEN | 4 | 1,28 |
| PEG-7 GLYCERYL COCOATE | 1 | 1 |
| PROPYLENE GLYCOL | 2 | 2 |
| GLYCERIN | 2 | 2 |
| CITRIC ACID | 0,25 | 0,25 |
| PEG-120 METHYL GLUCOSE DIOLEATE | 4,0 | 4 |
| AQUA | ad 100 | |

**Beispiel 2: Hautverträglichkeit einzelner Tenside**

Überraschenderweise wurde gefunden (Tab. 3), dass beim Test der reinen Tenside die nichtionogenen Vertreter sehr gute Ergebnisse erbrachten und zum Teil besser waren als der getestete Hautreiniger für Atopiker. Auch ein in einer vorherigen Messung getestetes Sodiumlaurylsulfosuccinat zeigte eine geringere Hautverträglichkeit als der Hautreiniger für Atopiker, obwohl es für seine gute Reinigungsleistung bei gleichzeitiger guter Hautverträglichkeit bekannt ist. Durch den Einsatz von Sodium Lauroyl Glutamate ließ sich die irritierende Wirkung der anionischen Tenside nicht auf das Niveau des Produktes für Atopiker senken.

**Tabelle 3**

| Tensid (INCI) | Rangfolge (RS) |
|---|---|
| Laureth-23 | 5 |
| Trideceth-10 | 5 |
| Oleth-10 | 9 |
| Hautreiniger für Atopiker | 9 |
| Ceteareth-23 | 14 |
| Laureth-6 | 15 |
| Laureth-7 | 23 |
| SLS | 37 |

(fortgesetzt)

| Tensid (INCI) | Rangfolge (RS) |
|---|---|
| Sodiumcocosulfat | 39 |

Insbesondere überraschten die guten Ergebnisse der nichtionogenen Tenside mit hohem Ethoxylierungsgrad, d.h. mit einer hohen Anzahl an Glykoleinheiten (hohes n in der Formel I).

**Beispiel 3: Hautverträglichkeit und Reinigungswirkung von Tensidkombinationen**

Es wurden Kombinationen verschiedener Tenside auf ihre Hautverträglichkeit getestet. In allen Irritationsstudien wurden Aktivgehalte der Tenside oder Tensidkombinationen von 8,5% getestetHierbei wurde gefunden, dass die Kombination einzelner Tenside mit schlechter Hautverträglichkeit, etwa des Sodiumcocosulfats, mit nichtionogenen Tensiden die Hautverträglichkeit erheblich verbesserte. Während reines Sodiumcocosulfat bei einem Aktivgehalt von 8,5% eine mit SLS (Aktivgehalt 0,5%) vergleichbare Hautirritation verursachte (Tab. 3), verbesserte sich diese durch die Kombination mit Trideceth-10 oder Laureth-23 auf das Niveau des Hautreinigers für Atopiker (Tab. 4).

**Tabelle 4**

| Tensid I (INCI), Aktivgehalt [%] | Tensid II (INCI), Aktivgehalt [%] | Tensid III (INCI), Aktivgehalt [%] | Rangfolge (RS) | Reinigungswirkung |
|---|---|---|---|---|
| Laureth-23, 8,5 | | | 5 | 2 |
| Laureth-23, 6 | Cocamidopropyl Betaine, 2,5 | | 13 | 2 |
| Laureth-23, 6 | Disodium Laureth Sulfosuccinate, 2,5 | | 19 | nicht bestimmt |
| Laureth-23, 6 | Disodium Laureth Sulfosuccinate, 6 | | 18 | 2 |
| Laureth-23, 6 | Cocamidopropyl Betaine, 3 | Disodium Laureth Sulfosuccinate, 3 | 12 | 1 |
| Hautreiniger für Atopiker | | | 17 | 3 |
| Laureth-23, 6 | Sodium Cocosulfate, 2,5 | | 18 | 2 |
| Sodium Cocosulfate, 6 | Trideceth-10, 2,5 | | 33 | 2 |
| Sodium Cocosulfate, 6 | Disodium Cocoamphoacetate, 2,5 | | 42 | 3 |
| SLS, 0,5 | | | 42 | nicht bestimmt |

Ein gegenüber dem Hautreiniger für Atopiker verbesserter Wert ergab sich auch für die Kombination von Laureth-23 mit Cocamidopropyl Betaine. Ein mit dieser Kombination durchgeführtes Screening ergab eine hervorragende Reinigungswirkung der Kombination dieser beiden Tenside mit Disodium Laureth Sulfosuccinate bei gleichbleibender Hautverträglichkeit trotz Erhöhung des Gesamtaktivgehaltes.

**Beispiel 4: Vergleich der synergistischen Wirkung von Laureth-23 mit weiteren Tensiden**

**Tabelle 5**

| **Tensid I (INCI), Aktivgehalt [%]** | **Cotenside (INCI), Aktivgehlt [%]** | **weitere Bestandteile, Aktivgehalt [%]** | **Rangfolge (RS)** | **Rangfolge RIT** | **Reinigungswirkung** |
|---|---|---|---|---|---|
| Laureth-23, 6 | Cocamidopropyl Betaine, 3 Disodium Laureth Sulfosuccinate, 3 | - | 12 | 14 | 1 |
| Laureth-23, 6 | Cocamidopropyl Betaine, 3,7 Disodium Laureth Sulfosuccinate, 3 | Coco Glycoside, 1 Glyceryl Oleate, n.a.* | | 12 | |
| Laureth-23, 6 | SLES, 6 | Coco Glycoside, 1 Glyceryl Oleate, n.a. | 38 | | |
| * n.a. = nicht anwendbar, da kein Tensid | | | | | |

Durch den Einsatz nichtionogener Tenside wie Laureth-23 ließ sich der Gesamtaktivgehalt bei der Verwendung von geeigneten Cotensiden wie Cocamidopropyl Betaine und Disodium Laureth Sulfosuccinate ohne Beeinträchtigung der Hautverträglichkeit erhöhen: ein erhöhter Aktivgehalt an Cocamidopropyl Betainen und von Coco Glycoside und Glyceryl Oleate ergab eine leicht verbesserte Hautverträglichkeit. Im Gegensatz hierzu zeigte sich jedoch bei Sodium Laurethsulfate (SLES) eine deutlich geringere Hautverträglichkeit.

**Beispiel 5: Vergleich Hautreiniger für Atopiker und Tensidkombination**

Die Reinigungswirkungen des Hautreinigers für Atopiker und der Kombination aus Laureth-23, Cocamidopropyl Betaine und Disodium Laureth Sulfosuccinate bei verschiedenen Aktivgehalten wurden verglichen. Es ergab sich eine stark verbesserte Reinigungswirkung dieser sehr hautverträglichen Tensidmischung. Die Zusammensetzungen enthielten neben Wasser 0,7 % Verdickungsmittel. (Tab. 6).

**Tabelle 6**

| **Tensid I (INCI), Aktivgehalt [%]** | **Tensid II (INCI), Aktivgehalt [%]** | **Tensid III (INCI), Aktivgehalt [%]** | **Aktivgehalt [%]** | **Reinigungswirkung [%]** |
|---|---|---|---|---|
| Laureth-23, 3 | Cocamidopropyl Betaine, 1,5 | Disodium Laureth Sulfosuccinate, 1, 5 | 6 | 50,41 |
| Laureth-23 4 | Cocamidopropyl Betaine, 2 | Disodium Laureth Sulfosuccinate, 2 | 8 | 56,71 |
| Laureth-23 6 | Cocamidopropyl Betaine, 3 | Disodium Laureth Sulfosuccinate, 3 | 12 | 58,75 |
| Hautreiniger für Atopiker | | | 13 | 38,24 |

## Patentansprüche

**1.** Hautreinigungsmittel enthaltend

- nichtionogene Tenside mit der allgemeinen Formel I

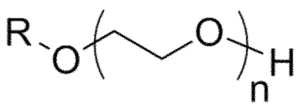

wobei n 5 bis 50 beträgt,
R eine lineare oder verzweigte, substituierte oder unsubstituierte, gesättigte oder ungesättigte Kohlenwasserstoffkette mit insgesamt 5 bis 40 C-Atomen ist und für nichtionogene Tenside mit identischem n identisch oder unterschiedlich sein kann, und
die Menge aller nichtionogenen Tenside mit der allgemeinen Formel I 2 bis 40 Gew.-%, bezogen auf die Gesamtmenge des Hautreinigungsmittels, beträgt,

- Cotenside ausgewählt aus Kombinationen von Sulfosuccinaten und Betainen, wobei die Menge aller Cotenside 2 bis 40 Gew.-%, berechnet ohne Gegenionen bezogen auf die Gesamtmenge des Hautreinigungsmittels, beträgt,
- Verdickungsmittel, wobei die Menge aller Verdickungsmittel 0,1 bis 20 Gew.-%, bezogen auf die Gesamtmenge des Hautreinigungsmittels, beträgt,
- Wasser,
- wobei das Betain eine quartäre Ammoniumgruppe, ein Carboxylat und einen unpolaren Teil umfasst.

**2.** Hautreinigungsmittel gemäß Anspruch 1, wobei die Menge aller nichtionogenen Tenside mit der allgemeinen Formel I 2 bis 20 Gew.-%, bevorzugt 2 bis 15 Gew.-%, mehr bevorzugt 2 bis 10 Gew.-%, noch mehr bevorzugt 3 bis 9 Gew.-%, jeweils bezogen auf die Gesamtmenge an Hautreinigungsmittel, beträgt.

**3.** Hautreinigungsmittel gemäß einem der Ansprüche 1 oder 2, wobei die Menge aller Cotenside 2 bis 20 Gew.-%, bevorzugt 2 bis 15 Gew.-%, mehr bevorzugt 2 bis 10 Gew.-%, noch mehr bevorzugt 2 bis 7 Gew.-%, berechnet jeweils ohne Gegenionen bezogen auf die Gesamtmenge an Hautreinigungsmittel, beträgt.

**4.** Hautreinigungsmittel gemäß einem der Ansprüche 1 bis 3, wobei das Hautreinigungsmittel eine Gesamtmenge an nichtionogenen Tensiden der allgemeinen Formel I und Cotensiden von 4 bis 25 Gew.-%, bevorzugt 4 bis 20 Gew.-%, mehr bevorzugt 5 bis 16 Gew.-%, jeweils berechnet ohne Gegenionen bezogen auf die Gesamtmenge des Hautreinigungsmittels, aufweist.

**5.** Hautreinigungsmittel gemäß einem der Ansprüche 1 bis 4, wobei R 8 bis 30, bevorzugt 10 bis 24, am meisten bevorzugt 12 bis 18 C-Atome aufweist.

**6.** Hautreinigungsmittel gemäß einem der Ansprüche 1 bis 5, wobei n 8 bis 50, bevorzugt 10 bis 40, mehr bevorzugt 14 bis 30, am meisten bevorzugt 15 bis 25 beträgt.

**7.** Hautreinigungsmittel gemäß einem der Ansprüche 1 bis 6, wobei R eine ungesättigte oder verzweigte Kohlenwasserstoffkette ist.

**8.** Hautreinigungsmittel gemäß einem der Ansprüche 1 bis 6, wobei R eine gesättigte, unverzweigte Kohlenwasserstoffkette ist, bevorzugt wobei R eine gesättigte, unverzweigte Kohlenwasserstoffkette mit 10 bis 24 C-Atomen ist, mehr bevorzugt wobei R eine gesättigte, unverzweigte Kohlenwasserstoffkette mit 12 - 18 C-Atomen ist, am meisten bevorzugt wobei das nichtionogene Tensid Laureth-23 ist.

**9.** Hautreinigungsmittel gemäß einem der Ansprüche 1 bis 8, weiterhin enthaltend Reibemittel, wie Polyethylenpartikel, Polyurethanpartikel, Silikate, Biopolymere, Bimsmehl, Kork, Glaskörper, Wachskörper, natürliche Kerne, Kernmehle wie Olivenkernmehle, Schalenmehle, Holzmehle, Lignin, Ligninderivate, Cellulose, Cellulosederivate oder Kombinationen daraus.

**10.** Hauteinigungsmittel gemäß einem der Ansprüche 1 bis 9, weiterhin enthaltend Überfettungsmittel, z.B. Glyceryl Oleate, Cetyl Palmitate, Lanolin, natürliche Pflanzenöle, Squalene oder Mischungen von Alkyl- und/oder Alkenyloligoglykosiden und Partialglyceriden, oder Kombinationen daraus.

**11.** Hautreinigungsmittel gemäß einem der Ansprüche 1 bis 10, weiterhin enthaltend Feuchthaltemittel (NMF), z.B.

Allantoin, Aminosäuren, Glycerin, Polyole, Harnstoff, Hyaluronsäure, Lactate, Lysozym, Serin, Vitamin A, Vitamin B, Vitamin C, hydrolysierte Pflanzenproteine, D-Panthenol oder Kombinationen daraus.

**12.** Hautreinigungsmittel gemäß einem der Ansprüche 1 bis 11, wobei das Verdickungsmittel Bentonite, Xanthane, Acrylate, Alginate, Cellulose-Ether, Carrageen, oder Kombinationen daraus umfasst.

**13.** Hautreinigungsmittel gemäß einem der Ansprüche 1 bis 12, wobei das Hautreinigungsmittel eine Viskosität bei 25 °C von 1000 bis 50000 mPas aufweist.

**14.** Hautreinigungsmittel gemäß einem der Ansprüche 1 bis 13, wobei das Hautreinigungsmittel bei einer Gesamtmenge an nichtionogenen Tensiden und Cotensiden von 8 Gew.-%, ohne Gegenionen bezogen auf die Gesamtmenge des Hautreinigungsmittels, einen Reinigungsfaktor von mindestens 50%, bevorzugt mindestens 55%, mehr bevorzugt mindestens 58%, am meisten bevorzugt mindestens 60%, aufweist, gemessen anhand einer Verschmutzung mit Modellschmutz bestehend (nach INCI) aus

- 54,0 Gew.-% Paraffinum Liquidum
- 18,1 Gew.-% Petrolatum
- 18,1 Gew.-% Lanolin
- 5,4 Gew.-% CI 77266 (Lampenruß)
- 3,6 Gew.-% CI77266 (Graphit)
- 0,8 Gew.-% CI 77499 (Eisenoxid),

jeweils bezogen auf die Gesamtmenge des Modellschmutzes, und anhand einer Hautwaschung mit dem Osnabrücker Hautwaschapparat gemäß Sonsmann FK, et al. Standardization of skin cleansing in vivo: part I. Development of an Automated Cleansing Device (ACiD) Skin Res Technol 2014, 20, 228-38.

**15.** Verwendung eines Hautreinigungsmittels gemäß einem der Ansprüche 1 bis 14 zur Hautreinigung.

## Claims

**1.** A skin cleaning agent, comprising

- non-ionogenic surfactants of the general formula I:

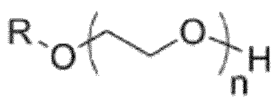

,

wherein n is from 5 to 50,
R is a linear or branched, substituted or unsubstituted, saturated or unsaturated hydrocarbon chain with a total of 5 to 40 carbon atoms, and may be identical or different for non-ionogenic surfactants with identical n, and
the amount of all non-ionogenic surfactants of the general formula I is from 2 to 40% by weight, based on the total amount of the skin cleaning agent,

- cosurfactants selected from combinations of sulfosuccinates and betaines, wherein the amount of all cosurfactants is from 2 to 40% by weight, calculated without counter ions and based on the total amount of the skin cleaning agent,
- thickening agents, wherein the amount of all thickening agents is from 0.1 to 20% by weight, based on the total amount of the skin cleaning agent,
- water,
- wherein said betaine includes a quaternary ammonium group, a carboxylate, and a non-polar part.

**2.** The skin cleaning agent according to claim 1, wherein the amount of all non-ionogenic surfactants of the general formula I is from 2 to 20% by weight, preferably from 2 to 15% by weight, more preferably from 2 to 10% by weight, even more preferably from 3 to 9% by weight, respectively based on the total amount of the skin cleaning agent.

3. The skin cleaning agent according to either of claims 1 or 2, wherein the amount of all cosurfactants is from 2 to 20% by weight, preferably from 2 to 15% by weight, more preferably from 2 to 10% by weight, even more preferably from 2 to 7% by weight, respectively calculated without counter ions and based on the total amount of the skin cleaning agent.

4. The skin cleaning agent according to any of claims 1 to 3, wherein said skin cleaning agent has a total amount of non-ionogenic surfactants of the general formula I and cosurfactants of from 4 to 25% by weight, preferably from 4 to 20% by weight, more preferably from 5 to 16% by weight, respectively calculated without counter ions and based on the total amount of the skin cleaning agent.

5. The skin cleaning agent according to any of claims 1 to 4, wherein R has from 8 to 30, preferably from 10 to 24, most preferably from 12 to 18, carbon atoms.

6. The skin cleaning agent according to any of claims 1 to 5, wherein n is from 8 to 50, preferably from 10 to 40, more preferably from 14 to 30, most preferably from 15 to 25.

7. The skin cleaning agent according to any of claims 1 to 6, wherein R is an unsaturated or branched hydrocarbon chain.

8. The skin cleaning agent according to any of claims 1 to 6, wherein R is a saturated unbranched hydrocarbon chain, preferably wherein R is a saturated unbranched hydrocarbon chain having from 10 to 24 carbon atoms, more preferably wherein R is a saturated unbranched hydrocarbon chain having from 12 to 18 carbon atoms, most preferably wherein said non-ionogenic surfactant is laureth-23.

9. The skin cleaning agent according to any of claims 1 to 8, further comprising rubbing agents, such as polyethylene particles, polyurethane particles, silicates, biopolymers, pumice powder, cork, glass bodies, wax bodies, natural kernels, kernel flours, such as olive stone flours, shell flours, wood flours, lignin, lignin derivatives, cellulose, cellulose derivatives, or combinations thereof.

10. The skin cleaning agent according to any of claims 1 to 9, further comprising superfatting agents, e.g., glyceryl oleate, cetyl palmitate, lanolin, natural vegetable oils, squalenes, or mixtures of alkyl and/or alkenyl oligoglycosides, and partial glycerides, or combinations thereof.

11. The skin cleaning agent according to any of claims 1 to 10, further comprising humectants (NMF), e.g., allantoin, amino acids, glycerol, polyols, urea, hyaluronic acid, lactates, lysozyme, serine, vitamin A, vitamin B, vitamin C, hydrolyzed vegetable proteins, D-panthenol, or combinations thereof.

12. The skin cleaning agent according to any of claims 1 to 11, wherein said thickening agent includes bentonites, xanthan gums, acrylates, alginates, cellulose ethers, carrageenan, or combinations thereof.

13. The skin cleaning agent according to any of claims 1 to 12, wherein said skin cleaning agent has a viscosity at 25 °C of from 1,000 to 50,000 mPa·s.

14. The skin cleaning agent according to any of claims 1 to 13, wherein said skin cleaning agent, with a total amount of non-ionogenic surfactants and cosurfactants of 8% by weight, without counter ions and based on the total amount of the skin cleaning agent, has a cleaning factor of at least 50%, preferably at least 55%, more preferably at least 58%, most preferably at least 60%, as measured on the basis of soiling with model soil consisting (according to INCI) of

- 54.0% by weight liquid paraffinum liquidum
- 18.1% by weight petrolatum
- 18.1% by weight lanolin
- 5.4% by weight CI 77266 (lampblack)
- 3.6% by weight CI 77266 (graphite)
- 0.8% by weight CI 77499 (iron oxide),

respectively based on the total amount of the model soil, and based on skin washing with the skin washing apparatus of the Osnabrück University according to Sonsmann F.K. et al., Standardization of skin cleansing in vivo: Part I. Development of an Automated Cleansing Device (ACiD), Skin Res Technol 2014, 20, 228-38.

**15.** Use of a skin cleaning agent according to any of claims 1 to 14 for cleaning the skin.

**Revendications**

**1.** Produit de nettoyage de la peau contenant

des tensioactifs non ionogènes de formule générale I

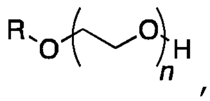

dans lequel n vaut de 5 à 50,
R est une chaîne d'hydrocarbure linéaire ou ramifiée, substituée ou non substituée, saturée ou insaturée avec un total de 5 à 40 atomes de carbone et peut être identique ou différentes pour des tensioactifs non ionogènes avec n identique, et
la quantité de tous les tensioactifs non ionogènes de formule générale I vaut de 2 à 40 % (m/m) par rapport à la quantité totale du produit de nettoyage de la peau,

- des co-tensioactifs sélectionnés parmi des combinaisons de sulfosuccinates et bétaïnes, dans lequel la quantité de tous les co-tensioactifs vaut de 2 à 40 % (m/m), calculée sans les contre-ions, par rapport à la quantité totale du produit de nettoyage de la peau,
- des agents épaississants, dans lequel la quantité de tous les agents épaississants vaut de 0,1 à 20 % (m/m) par rapport à la quantité totale du produit de nettoyage de la peau,
- de l'eau,
- la bétaïne comprenant un groupe ammonium quaternaire, un carboxylate et une partie non polaire.

**2.** Produit de nettoyage de la peau selon la revendication 1, dans lequel la quantité de tous les tensioactifs non ionogènes de formule générale I vaut de 2 à 20 % (m/m), de préférence 2 à 15 % (m/m), plus particulièrement 2 à 10 % (m/m), tout particulièrement 3 à 9 % (m/m), respectivement par rapport à la quantité totale du produit de nettoyage de la peau.

**3.** Produit de nettoyage de la peau selon l'une des revendications 1 ou 2, dans lequel la quantité de tous les co-tensioactifs vaut de 2 à 20 % (m/m), de préférence 2 à 15 % (m/m), plus particulièrement 2 à 10 % (m/m), tout particulièrement 2 à 7 % (m/m), calculée respectivement sans les contre-ions, par rapport à la quantité totale du produit de nettoyage de la peau.

**4.** Produit de nettoyage de la peau selon l'une des revendications 1 à 3, dans lequel le produit de nettoyage de la peau comporte une quantité totale de tensioactifs non ionogènes de formule générale I et de co-tensioactifs de 4 à 25 % (m/m), de préférence 4 à 20 % (m/m), plus particulièrement 5 à 16 % (m/m), calculée respectivement sans les contre-ions, par rapport à la quantité totale du produit de nettoyage de la peau.

**5.** Produit de nettoyage de la peau selon l'une des revendications 1 à 4, dans lequel R comporte 8 à 30, de préférence 10 à 24, tout particulièrement 12 à 18 atomes de carbone.

**6.** Produit de nettoyage de la peau selon l'une des revendications 1 à 5, dans lequel n vaut entre 8 et 50, de préférence 10 à 40, plus particulièrement 14 à 30, tout particulièrement 15 à 25.

**7.** Produit de nettoyage de la peau selon l'une des revendications 1 à 6, dans lequel R est une chaîne d'hydrocarbure insaturée ou ramifiée.

**8.** Produit de nettoyage de la peau selon l'une des revendications 1 à 6, dans lequel R est une chaîne d'hydrocarbure saturée non ramifiée, de préférence dans lequel R est une chaîne d'hydrocarbure saturée non ramifiée avec 10 à 24 atomes de carbone, plus particulièrement dans lequel R est une chaîne d'hydrocarbure saturée non ramifiée avec 12 à 18 atomes de carbone, tout particulièrement dans lequel le tensioactif non ionogène est le laureth-23.

**9.** Produit de nettoyage de la peau selon l'une des revendications 1 à 8, contenant en outre des agents abrasifs comme des particules de polyéthylène, des particules de polyuréthane, des silicates, des biopolymères, de la pierre ponce pulvérisée, du liège, des corps vitreux, des corps cireux, des graines naturelles, des poudres de noyaux telles que poudres de noyaux d'olive, de coquillages, de bois, de la lignine, des dérivés de lignine, de la cellulose, des dérivés de cellulose ou des combinaisons de ceux-ci.

**10.** Produit de nettoyage de la peau selon l'une des revendications 1 à 9, contenant en outre des agents surgraissants, par exemple de l'oléate de glycéryle, du palmitate de cétyle, de la lanoline, des huiles végétales naturelles, du squalène ou des mélanges d'alkyl et/ou d'alcényl glycosides et des glycérides partiels, ou des combinaisons de ceux-ci.

**11.** Produit de nettoyage de la peau selon l'une des revendications 1 à 10, contenant en outre des agents humectants, par exemple de l'allantoïne, des acides aminés, du glycérol, des polyols, de l'urée, de l'acide hyaluronique, des lactates, du lysozyme, de la sérine, de la vitamine A, de la vitamine B, de la vitamine C, des protéines végétales hydrolysées, du D-panthénol ou des combinaisons de ceux-ci.

**12.** Produit de nettoyage de la peau selon l'une des revendications 1 à 11, dans lequel l'agent épaississant comprend de la bentonite, du xanthane, des acrylates, des alginates, des éthers de cellulose, des carraghénanes ou des combinaisons de ceux-ci.

**13.** Produit de nettoyage de la peau selon l'une des revendications 1 à 12, dans lequel le produit de nettoyage de la peau comporte une viscosité à 25 °C de 1 000 à 50 000 mPas.

**14.** Produit de nettoyage de la peau selon l'une des revendications 1 à 13, dans lequel le produit de nettoyage de la peau comporte, pour une quantité totale de tensioactifs non ionogènes et de co-tensioactifs de 8 % (m/m), sans les contre-ions, par rapport à la quantité totale du produit de nettoyage de la peau, un facteur de nettoyage d'au moins 50 %, de préférence au moins 55 %, plus particulièrement au moins 58 %, tout particulièrement au moins 60 %, mesuré à l'aide d'une salissure avec une tache modèle constituée (selon l'INCI) de

- 54,0 % (m/m) de Paraffinum Liquidum
- 18,1 % (m/m) de vaseline (Petrolatum)
- 18,1 % (m/m) de lanoline
- 5,4 % (m/m) de CI 77266 (noir de fumée)
- 3,6 % (m/m) de CI 77266 (graphite)
- 0,8 % (m/m) de CI 77499 (oxyde de fer),

respectivement par rapport à la quantité totale de la tache modèle, et à l'aide d'un lavage cutané avec l'appareil de lavage cutané d'Osnabrück selon Sonsmann FK, et al. Standardization of skin cleansing in vivo: part I. Development of an Automated Cleansing Device (ACiD) Skin Res Technol 2014, 20, 228-38.

**15.** Utilisation d'un produit de nettoyage de la peau selon l'une des revendications 1 à 14 pour le nettoyage de la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4038076 A1
- DE 19748921 A1
- EP 1152051 A2
- US 20160100574 A1
- WO 2005051341 A2


### In der Beschreibung aufgeführte Nicht-Patentliteratur


- **SONSMANN FK et al.** Standardization of skin cleansing in vivo: part I. Development of an Automated Cleansing Device (ACiD). *Skin Res Technol,* 2014, vol. 20, 228-38
- **SCHRADER K ; ROHR M.** Methods for measuring the skin-cleansing effect of surfactants in comparison with skin roughness and compatibility. *Clin Dermatol.,* 1996, vol. 14 (1), 57-65
- **SONSMANN FK ; STRUNK M ; GEDIGA K et al.** Standardization of skin cleansing in vivo: part I. Development of an Automated Cleansing Device (ACiD). *Skin Res Technol,* 2014, vol. 20, 228-38